# EUROPEAN PATENT APPLICATION

(11) **EP 4 768 126 A2**
(43) Date of publication of application: **01.07.2026**
(21) Application number: 26176675.2
(22) Date of filing: 03.05.2022
(51) Int. Cl.: B01L 7/00

(54) **SYSTEMS, METHODS, AND APPARATUS FOR AUTOMATED SELF-CONTAINED BIOLOGICAL ANALYSIS**

(30) Priority: 03.05.2021 US 202163183492 P
(62) Divisional of application: 22799430.8
(71) Applicant: Co-Diagnostics, Inc., Salt Lake City UT 84109 (US)
(72) Inventor: ABBOTT, Richard, David, Draper, UT 84020 (US); NIELSEN, David, Sandy, UT 84093 (US); JONES, Connor, Ogden, UT 84403 (US); PORITZ, Mark, Aaron, Salt Lake City, UT 84103 (US); RIRIE, Kirk, M., Salt Lake City, UT 84108 (US); THURSTON, Christopher, M., Orlando, FL 32819 (US); DAVID, Derek, Salt Lake City, UT 84121 (US); SANDSTROM, Craig, Sandy, UT 84070 (US)
(74) Representative: Forresters IP LLP

(57) **Abstract**

Systems, apparatus, and methods for conducting amplification-based analyses, including PCR testing. In one illustrative embodiment, a system may include a testing container assembly and a testing unit. The testing container assembly may include a sample collection port, a sample preparation chamber, and a reaction chamber. The sample collection port may include a bottom opening sealed by a plug member. In use, the testing container assembly may be placed in a seat of the testing unit with a sample in the sample collection port, closed by a lid. A plunger may dislodge the plug member and the sample drawn into the sample preparation chamber. Once sample preparation is complete, a channel may be opened, and the prepared sample flows into the reaction chamber which is then sealed. Testing including amplification reactions, may then be performed, followed by detection, as by detecting fluorescent emissions in the reaction chamber.

## Description

### TECHNICAL FIELD

This disclosure relates to systems, methods, and apparatus for conducting biological analyses, including PCR testing to detect organisms.

### BACKGROUND

The polymerase chain reaction (PCR) has become a method of choice for sensitive and specific detection of pathogens in a sample. The detection of nucleic acids derived from pathogens by PCR is currently the authoritative method for the diagnosis of many infectious diseases. One challenge arising during the COVID-19 pandemic has been the ability to perform such analyses quickly on a wide community scale, with near real-time communication of results, such that the detection of cases, contract tracing, and surveillance can be performed to effectively contain and control the disease. A number of PCR-based testing platforms currently exist that provide closed sample-to-answer designs and require minimum operator intervention with a one-to-two-hour turn-around time. But these designs involve complex mechanics and consumables making them expensive, thus limiting their placements mainly to clinical labs, healthcare providers, government facilities, and other professional organizations. Other PCR systems allow consumers to collect their own sample, but the actual testing and analysis are performed at a centralized lab to where the samples are shipped, and time-to-result often exceeds 24 hours.

A system or process for performing a biological analysis that is simple to operate would be an improvement in the art. Such a system that requires minimal sample processing for use would be further improvement in the art.

### SUMMARY

The present disclosure is directed to systems, apparatus, and methods for conducting amplification-based analyses, including PCR testing. In one illustrative embodiment of a first aspect of the present disclosure, a system may include a testing container assembly and a testing unit with a seat for receiving the testing container assembly. In some embodiments, the testing container assembly may be formed as a single use disposable unit.

The testing container assembly may include a sample collection port, a sample preparation chamber, and a reaction chamber. The sample collection port may be formed as a funnel-shaped cup member with a bottom opening that is scaled by a plug member. The sample preparation chamber may be maintained under vacuum and in fluid connection with the bottom opening. The reaction chamber may similarly be maintained under vacuum. The sample preparation chamber and the reaction chamber may each contain any required reaction components. The testing unit may include components that align with the various chambers of the testing container assembly and enable the analysis to be performed

In another aspect of the present disclosure, illustrative methods of analyzing a sample are provided, and may include providing a testing container assembly and placing the sample in the collection port thereof, which is then closed with a lid assembly, which may include a movable plunger. The testing container assembly may be placed in the seat of the testing unit. The plunger may dislodge the plug member, allowing the sample to be drawn into the sample preparation chamber by the vacuum. In some embodiments, this may be accomplished by securing the lid assembly to the collection port. In others, the plunger may be advanced to dislodge the plug. A temperature control device in conductive contact with the sample preparation chamber may be actuated to perform or terminate sample preparation reactions. A channel in communication with the reaction chamber may then be opened, to allow prepared sample to flow into the reaction chamber. The reaction chamber may then be sealed and a reaction, such as PCR, is performed, as by actuating a temperature control device in conductive contact with the reaction chamber. During and/or after the reaction, detection may take place, as by detecting a fluorescent emission from a fluorescent dye in the reaction chamber that is actuated when a reaction product of interest is present. Melting curve analysis may optionally be performed during and/or after the reaction to confirm the presence or absence of specific analytes or specific variants of analytes. In some embodiments, the testing unit may include a laser as a light source and a multi-channel spectrometer may be used for detection.

In another aspect, a testing unit may include a CPU that controls its various components to perform a testing procedure, and a communications gateway, such as a Bluetooth or other wireless communications component. In some embodiment, the testing unit may communicate with a remote system that directs the particular assay requirements and receives and analyzes data collected by the testing unit to determine a result. In some embodiments the system may utilize a handheld device, such as a user's smartphone, to route communications and may use an app on the device to report results to a user.

In another aspect of the present disclosure, exemplary embodiments of systems, and containers in accordance with the present disclosure may be used with methods of detecting a nucleic acid in a sample. In such methods, a container or receptacle having a plurality of fluidly connected chambers including a sample preparation zone, and an amplification zone, with one or more sealable ports fluidly connecting the chambers may be provided. It will be appreciated that the sealable ports may provide the only access from an exterior of the receptacle to the chambers. The sample may be introduced into the first chamber via a collection port, wherein the collection port has a bottom opening sealed by a plug member. When a lid assembly with a movable plunger assembly is secured to the collection port, the plug member may be contacted with the plunger to dislodge the plug member allowing the sample benter a sample preparation chamber in fluid connection with the bottom opening.

Optionally, the sample may then be treated in the sample preparation chamber, as by heating to deactivate enzymes in the sample, or with reactants contained in the sample preparation chamber. A seal may be opened to allow the sample to flow from the sample preparation chamber to a reaction chamber. In some embodiments, this may be performed by advancing the plunger into the sample preparation chamber to pressure the sample and fracture the seal. The reaction chamber may then be sealed. In the reaction chamber, nucleic acids in the sample with may be mixed with PCR components including primers configured for amplifying one or more targets; and the target nucleic acids may be amplified.

A fluorescence emission signal from a fluorescent dye in the amplification zone may be detected, wherein fluorescence is excited by a laser diode, and emission is detected by a multi-channel spectrometer. A multi-channel spectrometer capable of processing at least 4 spectral channels in parallel in the wavelengths from approximately 350nm to 1000nm may be used. The fluorescence data may be communicated to a cloud or local CPU via a mobile phone or other user-controlled device or operating system. The data may then be analyzed, and the analysis result may be received from the cloud or local CPU by a mobile phone or other user-controlled device or operating system.

### DESCRIPTION OF THE DRAWINGS

It will be appreciated by those of ordinary skill in the art that the various drawings are for illustrative purposes only. The nature of the present disclosure, as well as other embodiments in accordance with this disclosure, may be more clearly understood by reference to the following detailed description, to the appended claims, and to the several drawings.
FIGS. 1A, 1B, 1C, 1D and 1E are respectively, perspective, bottom, side, top, and sectional views of a first illustrative embodiment of a body for a testing container assembly in accordance with the principles of the present disclosure.
FIG. 2 is a side view of an illustrative plug member for use with the testing container assembly of FIGS. 1A through 1E.
FIGS. 3A and 3B are bottom perspective and sectional side views of one illustrative lid member for use with the testing container assembly of FIGS. 1A through 1E.
FIGS. 4A and 4B are perspective and sectional side views of one illustrative plunger member for use with the testing container assembly of FIGS. 1A through 1E and the lid member of FIGS. 3A and 3B.
FIGS. 5A and 5B are perspective and sectional side views of the testing container assembly of FIGS. 1A through 1E with the lid member of FIGS. 3A and 3B and the plunger member of FIGS. 4A and 4B for use.
FIG. 6A is a partial cutaway side view of the testing container assembly of FIGS. 1A through 1E in position in the seat of one illustrative embodiment of a testing unit in accordance with the principles of the present disclosure.
FIG. 6B is across-sectional view depicting an illustrative embodiment of a detection assembly similar to that depicted in FIG. 6 which uses spherical lenses to focus illumination and collection of fluorescent signal.
FIGS. 6C is a perspective view of a second illustrative embodiment of a testing container in accordance with the principles of the present disclosure in position in the seat of a second illustrative embodiment of a testing unit in accordance with the principles of the present disclosure.
FIG. 6D is a section side view taken along line A-A of FIG. 6C.
FIG. 7 shows fluorescence at three wavelengths monitored in the testing unit in real time during amplification of a human genetic target using dried amplification reagents in the testing container assembly.
FIGS. 8A and 8B are results of amplicon melting that was performed in the amplification zone of the testing container assembly, where three-color fluorescence was monitored in real time during melting (FIG. 8A) and from which derivative melting curves were calculated and rendered (FIG. 8B) by an external CPU that was in communication with a testing unit in accordance with the principles of the present disclosure, where F is fluorescence, T is temperature, and dF/dT is the derivative.
FIG. 9 is a derivative melting curve calculated from fluorescence being monitored at six wavelengths simultaneously during melting in a testing unit using two double-stranded DNA fragments in a testing container assembly in accordance with the present disclosure.

### DETAILED DESCRIPTION

The present disclosure relates to apparatus, systems, and methods related to conducting biological analyses, including PCR testing. It will be appreciated by those skilled in the art that the embodiments herein described, while illustrative, are not intended to limit this disclosure or the scope of the appended claims. Those skilled in the art will also understand that various combinations or modifications of the embodiments presented herein can be made without departing from the scope of this disclosure. All such alternate embodiments are within the scope of the present disclosure.

The present disclosure is directed to systems, apparatus, and methods for conducting biological analyses, including PCR testing. It will be appreciated that while PCR is the amplification method used in the examples herein, it is understood that any amplification method that uses a primer may be suitable, whether the signal or target is amplified. In fact, any proximity-based amplification approaches known to those of skill in that art may be used, including assays for the signal amplification to detect antigens. Some suitable procedures may include polymerase chain reaction (PCR); strand displacement amplification (SDA); nucleic acid sequence-based amplification (NASBA); cascade rolling circle amplification (CRCA), loop-mediated isothermal amplification of DNA (LAMP); isothermal and chimeric primer-initiated amplification of nucleic acids (ICAN); target basedhelicase dependent amplification (HDA); transcription-mediated amplification (TMA), CRISPR-Cas9-triggered strand displacement amplification, immuno-PCR and the like. Amplification methods may further include analyses such as melting curve analysis. Therefore, when the term PCR is used, it should be understood to include other alternative amplification methods and analysis of amplification products. It is understood that protocols may need to be adjusted accordingly.

For purposes of understanding, the systems and methods herein will be discussed in connection with an illustrative PCR-based analysis for coronavirus in a saliva sample or a nasal swab sample, such as a COVID-19 test for human. It will be appreciated that different assays for different conditions using various samples and differing reagents and reaction parameters may be used. Environmental and agricultural samples containing possible biological materials may also be used. All such variations are within the scope of the present disclosure.

In one illustrative embodiment, a system in accordance with the present invention may include a testing container and a testing unit with a seat for receiving the testing container assembly. In some embodiments, the testing container assembly may be formed as a single use disposable unit.

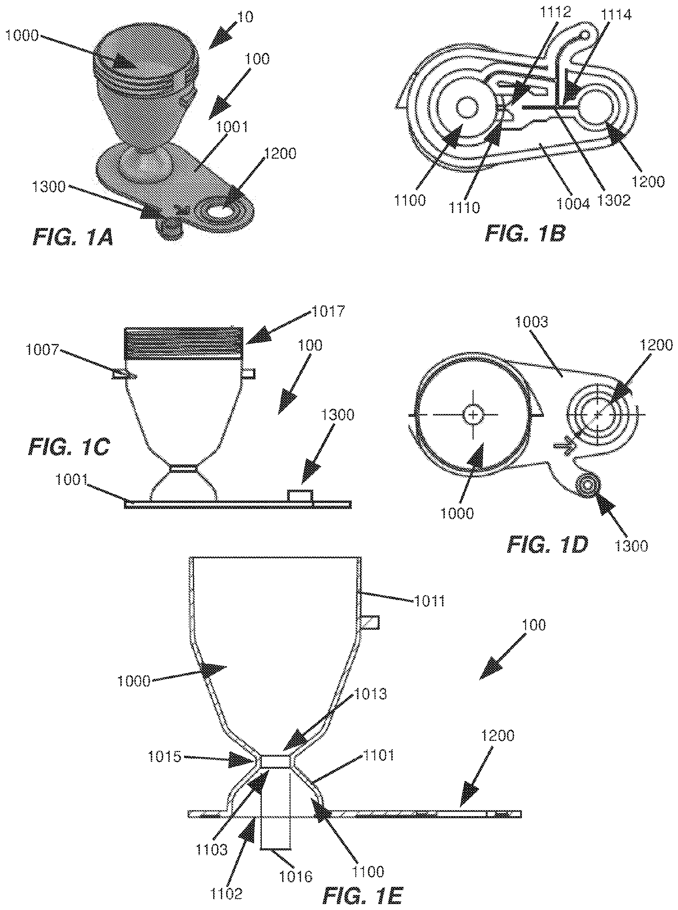

Turning to FIGS. 1A, 1B, 1C, 1D and 1E, one exemplary testing container assembly generally indicated at 10 is depicted. It will be appreciated that the term testing container assembly, as used herein refers to an assembly including one or more receptacles or enclosures for containing a sample and reactants for processing in accordance with this disclosure and is used interchangeably with the term container assembly. In the depicted embodiment, the testing container assembly 10 may include a body 100 formed of a generally rigid material that defines a sample collection port 1000, a sample preparation chamber 1100 and a reaction chamber 1200. It will be appreciated that the body 100 may be constructed from a material that is compatible with the particular reactions and assays being conducted with a particular embodiment. For example, injection moldable polymeric materials that are non-reactive with PCR reactants and substrates may be used. In the depicted embodiment, a base 1001 may be formed as generally planar member with an upper surface 1003 and an opposite lower surface 1004.

Sample preparation chamber 1100 may be disposed in the base 1001 and defined by a sidewall 1101 that rises from the base 1001 to converge toward an upper opening 1103. At a lower end, the sample preparation chamber 1100 may have an open port 1102 that passes through the lower surface 1004.

At an upper end of the sample preparation chamber 1100, the sample collection port 1000 may be disposed. As depicted, sample collection port 1000 may be formed as a funnel-shaped cup defined by a surrounding sidewall 1011 that tapers to a bottom opening 1013. The sidewall 1011 may include a threaded upper portion 1017 or other structure allowing a lid to be secured thereto.

As depicted the bottom opening 1013 and upper opening 1103 may be aligned and define the upper ends of a bore 1015 defined by a surrounding sidewall.

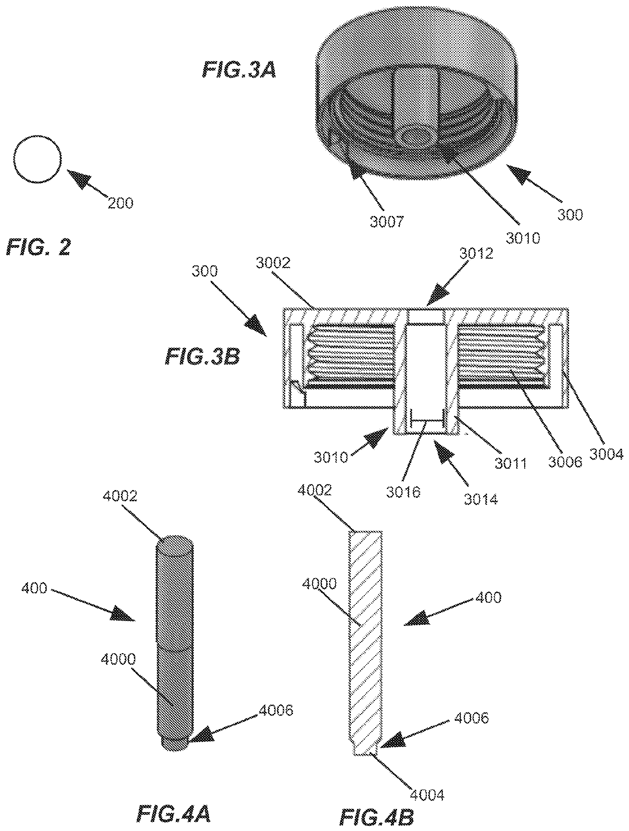

For use, the bore 1015 may contain a plug member that seals the bottom of sample collection port 1000 and the top of the sample preparation chamber 1100. As depicted in FIG. 2, the plug member may be a ball bearing 200 that is sized to correspond to the diameter 1016 of the bore 1015. In some embodiments, the ball bearing may have a diameter slightly larger than the diameter of the bore, with the material of the sidewall compressing the plug member to retain it in position. It will be appreciated that in some other embodiments, the plug member may have a different shape, or may be formed as a frangible plug across the bore 1015. In additional embodiments, the frangible seal may be a pierceable plug or seal provided in the bore 1015.

Reaction chamber 1200 may be formed as a bore that passes through the base 1001 at a location spaced apart from the sample preparation chamber 1100. An upper end of the reaction chamber 1200 may be sealed by a material that is sufficiently transparent to the wavelengths used in detecting the assays performed to serve as a detection window 1203. Detection window 1203 may be formed of a flexible material that allows the window to flex to form a domed shape when the chamber is filled.

The bottom surface 1004 of the base 1001 may include a series of channels formed in the lower surface 1004. As depicted, a reaction channel 1302 may lead from the sample preparation chamber 1100 to reaction chamber 1200. Other channels may lead from the chambers or channels to a vacuum port 1300.

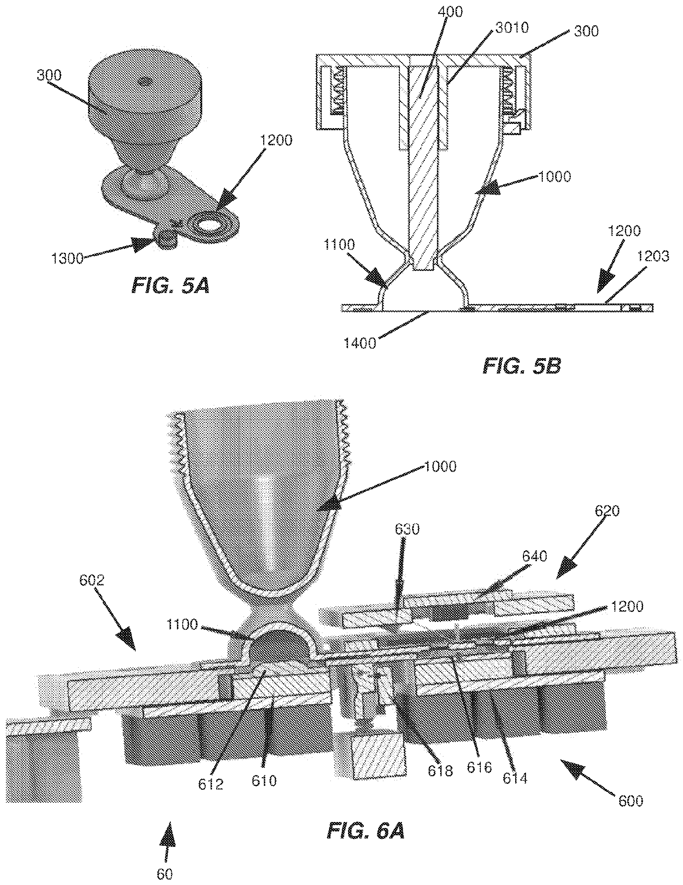

The bottom surface 1004 may be covered by a bottom seal sheet 1400 (FIG. 5B) of flexible material. The flexible material may be a flexible plastic film or other flexible material such as polyester, polyethylene terephthalate (PET), polycarbonate, polypropylene, polymethylmethacrylate, and mixtures thereof that can be made by any process known in the art, including extrusion, plasma deposition, and lamination. The sheet may be adhered by heat treatment or a suitable adhesive to the edges and/or other portions of the bottom surface but remain flexible in the areas where it covers sample preparation chamber 1100, reaction chamber 1200 and the various channels. It will be appreciated that in some embodiments, the detection window 1203 and bottom sheet 1400 may be formed from like materials. It will be appreciated that the bottom seal sheet 1400 may be constructed from a material that is compatible with the particular reactions and assays being conducted with a particular embodiment.

The sample preparation chamber 1100 and the reaction chamber 1200 may be maintained under vacuum. In some embodiments, vacuum may be applied using vacuum port 1300. In some embodiments, the application of vacuum may draw bottom seal sheet 1400 to the bottom surface 1004 and the contact may form a frangible seal between the bottom seal sheet 1400 and the bottom surface, that seals sample preparation chamber 1100 and the reaction chamber 1200 from one another. In other embodiments, a frangible seal between bottom seal sheet 1400 and bottom surface 1004 may be formed by the application of heat. It will be appreciated that any method of forming a frangible seal between the bottom seal sheet 1400 and the bottom surface known to those of skill in the art may be used. Vacuum port 1300 may then be sealed or may contain a seal member allowing the vacuum to be drawn. Vacuum port 1300 may be disposed on an extension protruding from base 101 or may be placed directly on base 1001.

As best depicted in FIG. 1B, the sample preparation chamber 1100 may include a stress riser feature that facilitates opening of the frangible seal, as discussed elsewhere herein. In the depicted embodiment, the sample preparation chamber 1100 includes an elongated section 1110 divided by the stress riser 1112, which appears as a portion of bottom surface tapering to a point that divides the elongates section into two "pockets". When the sample preparation chamber 1100 is pressurized, the pockets fill with sample and thereby expand and the tapered point serves as a starting point for the bottom seal sheet 1400 to peel away from bottom surface 1300 as the frangible seal is opened.

It will be appreciated that the sample collection port 1000, the sample preparation chamber 1100 and the reaction chamber 1200 may each contain any required reaction components, including reactants in dried form that are disposed in such chamber and sealed therein. Dried forms of reaction components can be prepared by a variety of known methods for lyophilization (for instance Babonneau et al. (2015) Development of a Dry-Reagent-Based qPCR to Facilitate the Diagnosis of Mycobacterium ulcerans Infection in Endemic Countries. PLoS Negl Trop Dis 9(4): e0003606. https://doi.org/10.1371/journal.pntd.0003606, and Panoka et al US. Pat.No. 8,900,525, the contents of each of which are incorporated by reference herein in their entirety) or for air drying (for instance Metzler et al US Pat. No. 8,652,811, and Rombach, et al, (2014) Real-time stability testing of airdried primers and fluorogenic hydrolysis probes stabilized by trehalose and xanthan, BioTechniques 57:151-155 . doi 10.2144/000114207 the contents of which are incorporated by reference herein in their entirety). For example, the sample collection port 1000 may contain reagents to initiate a sample preparation procedure in the form of a pellet or film disposed on the sidewall in a lower portion thereof The sample preparation chamber 1100 may similarly contain sample preparation components.

One potential reagent may be a protease, such as proteinase K which can be used to digest proteins in a human sample that might degrade DNA or RNA or otherwise inhibit PCR. Alternatively, or in addition, TCEP (tris(2-carboxyethyl) phosphine) or NAC (N-acetylcysteine) may be used to cleave the disulfide bonds present in saliva or nasal cavity enzymes and thus make it easier to denature them. It will be appreciated that the particular components and/or reagents may vary by the assay to be performed. In some embodiments, a mineral oil, paraffin wax, or a combination of mineral oil and paraffin wax may be present in the first chamber. During use, these may float up above the aqueous layer during a loading and/or heating step, to thereby reduce bubbles and ensure that the sample is closest to the heater. Paraffin wax may also act to plug the top outlet to seal the sample in the second chamber.

The reaction chamber 1200 may contain assay components and reactants, along with analysis components, such as dyes, colorants and the like. In some embodiments the top film sealing the reaction chamber 1200 may have enzymes (including polymerases or transcriptases), nucleotide sand/or buffers freeze-dried or air dried thereon. The lower sealing film may have primers, oligonucleotides and/or dyes air dried or freeze dried thereon. It will be appreciated that in some embodiments, the drying may be performed on the flexible films itself to deposit the materials in the appropriate place, while in others pre-dried materials maybe deposited in place.

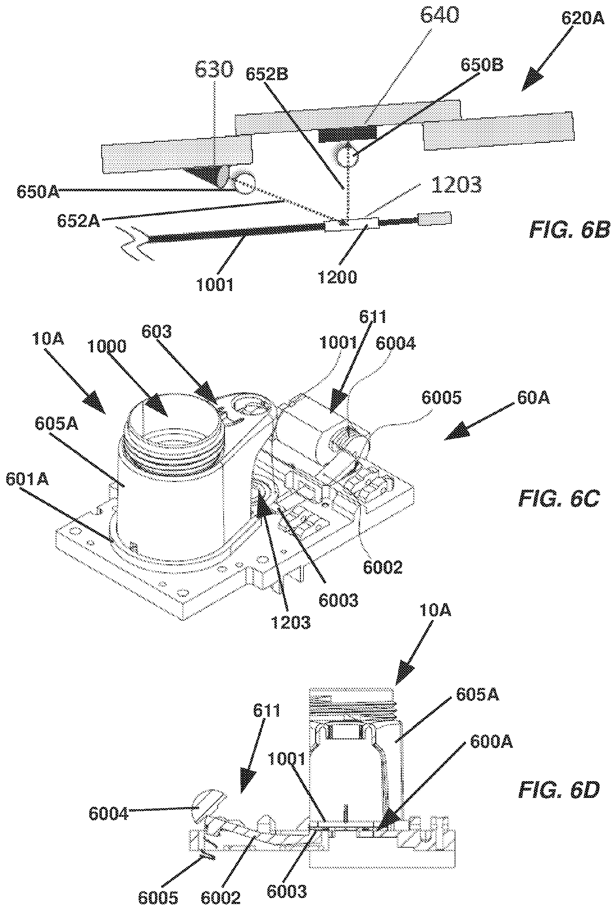

As depicted in FIGS. 6B and 6C, an outer shroud, 605A may be used to reinforce and protect the assembly 10 or 10A. Shroud 605A may include a body formed from a suitable material that can be secured to base 1001 and surround the sample collection port 1000 body, sample preparation chamber 1100 and a portion of the upper surface of base 1001 to facilitate handling during use and provide reinforcement. A locking mechanism 603 may be disposed on the shroud 605A at an upper surface to secure lid 300, as discussed further herein.

Turning to FIGS. 3A and 3B one illustrative lid member 300 for use with the testing container assembly of FIGS. 1A through 1E is depicted. In the depicted embodiment, the lid 300 is formed as a cap that attaches to the threaded upper portion 1017 of the sample collection port 1000, as depicted in FIGS. 5A and 5B, using threading 3006 disposed on the inner side of the sidewall 3004 such that the upper portion 3002 of the cap closes the open top of the sample collection port 1000. In some embodiments, one or more locking structures, such as a locking tab 3007 may connect to a corresponding locking structure 1007 on the sample collection port 1000 or on shroud 605A. It will be appreciated that this particular manner of closing is merely exemplary, and that any suitable arrangement known to those of skill in the art may be used.

A plunger bore 3010 may be disposed in the lid member 300. In the depicted embodiment, the plunger bore 3010 may be formed as an elongated tube 3011 that extends from the lower surface of the upper portion 3002 of the lid downwards to bottom opening 3014. A top opening 3012 in the upper portion 3002 of the cap similarly aligns with the bore 3010. Bore 3010 has a diameter indicated at 3016 that may be generally consistent from the upper portion 3002 of the cap down, with the portion passing through the upper portion 3002 of the cap narrowed to provide a stop for the plunger 400.

Plunger 400 is best depicted in FIGS. 4A and 4B. As depicted, the plunger 400 may be formed as shaft member 400, with a generally columnar shape having a planar upper end 4002 and a generally planar lower end 4004. The body of the shaft 4000 may include a lower portion with a smaller diameter to form a recess or step 4006. It will be appreciated that the particular size and shape of the plunger and the cap portion may vary in different embodiments in order to provide the functions discussed further herein.

FIGS. 5A and 5B depict the testing container assembly of FIGS. 1A through 1E with the lid member 300 and the plunger member 400 (FIG. 5B). As depicted, the larger diameter of the plunger body 4000 may correspond to the diameter of bore 1015, such that its sides seal against the bore 1015 with the smaller distal end passing into the reaction chamber 1200, when the lid 300 lid is secured to the sample collection port 1000. It will be appreciated that the plunger 400 may act in the manner of a syringe plunger to seal the bore 1015 and provide volume control to the sample preparation chamber 1100.

FIG. 6A depicts the testing container assembly 10 in position in a seat 600 in one exemplary embodiment of a testing unit 60 in accordance with the principles of the present disclosure. Seat 600 may be formed as a surface for receiving the base 1001 of the container assembly, with the components of the testing unit 600 aligned to allow an assay to be performed. Surface 602 may include alignment features, such as a tabs or recessed portions that allow the base to be properly aligned and maintained in position. Where components are disposed over portions the container assembly 10 during use, the seat 600 may be placed in a chamber of the unit 60, with such components movable as the unit 60 is opened or closed to allow a user to easily place the container assembly 10 in the seat 600. Seat 600 may receive the base 1001 in a position that is angled or tilted at least slightly away from a horizontal plane to expedite the movement of any air bubbles in a solution in the reaction chamber 1200 away from the center of detection window 1203.

Seat 600 may include a first temperature control element 610, which is in thermally conductive contact with sample preparation chamber 1100 for use. In the depicted embodiment, the first temperature control element is a Peltier element, and a conductive member 612 is disposed in contact therewith that aligns with the bottom of the sample preparation chamber 1100 when the container assembly 10 is placed in the seat 600. It will be appreciated that the depicted Peltier element is merely illustrative, and any temperature control assembly known to those of skill in the art may be used, including tubing for circulation of cooled or heated fluid, resistance heating elements, and the like.

Similarly, seat 600 may include a second temperature control element 614, which is in thermally conductive contact with reaction chamber 1200 for use. In the depicted embodiment, the second temperature control element is a Peltier element, and a conductive member 616 is disposed in contact therewith that aligns with the bottom of the reaction chamber 1200 when the container assembly 10 is placed in the seat 600. It will be appreciated that the depicted Peltier element is merely illustrative, and any temperature control assembly known to those of skill in the art may be used, including tubing for circulation of cooled or heated fluid, resistance heating elements, and the like.

A retractable sealing unit, such as a heat sealer 618 may be positioned in the seat 600 to reside underneath the reaction channel 1302 when the container assembly 10 is placed in the seat 600. The retractable sealing unit may be actuated to move upwards, and heat as required to melt the sealing sheet 1400 through the channel 1302 to thereby seal and isolate the reaction chamber 1200 as discussed further herein.

In the depicted embodiment, a detection assembly 620 may be positioned near the seat 600. Detection assembly 620 may include the components necessary for performing detection through detection window 1203, including an energy source and a sensor. Where detection of fluorescent emissions will be used, an energy source for excitation may be present. In the depicted embodiment, this energy source is a laser 630. Use of a laser allows for energy input near a single predefined wavelength, which can eliminate the need for waveguides and/or filters required in some known detection systems. One suitable laser assembly may be the PL-450B type laser, which is commercially available from OSRAM Opto Semiconductors Inc. which is a Blue Laser Diode in a metal can package which emits light at a wavelength of about 450 nm. It will be appreciated that any suitable laser may be used. Alternatively, an LED may also be used as a cost-effective option for an energy source in conjunction with the use of glass or bandpass/interference filters. The sensor may be an optical detector 640, such as a multi-channel spectrometer. One suitable sensor is the AS7341-DLGT, which is commercially available from AMS, which is an 11-channel spectrometer with a spectral response is defined in the wavelengths from approximately 350nm to 1000nm. It will be appreciated that any suitable sensor may be used. The use of a multi-channel spectrometer allows for emitted fluorescence at multiple wavelengths to be detected.

FIG. 6B depicts a cross section of another illustrative embodiment of a detection assembly 620A for a testing unit in accordance with the present disclosure, in relation to reaction chamber 1200 in the base 1001 of a container assembly positioned in a testing unit seat. Detection assembly 620A is similar to detection assembly 620 and may include the components necessary for performing detection through detection window 1203, including an energy source, such as a laser 630 and an optical detector, such as a multi-channel spectrometer 640. Additionally, a first spherical lens 650A may be positioned in front of laser 630 and a second spherical lens 650B may be positioned in front of detector 640 to focus the illumination and collection of fluorescent signal (depicted by dotted arrows 652A and 652B) through the detection window 1203 of reaction chamber 1200.

Spherical lenses 652A and 652B may be ball lenses, which may be formed as a transparent spherical ball. Suitable materials may include silica glass or another highly transparent material with a suitable index of refraction to allow use of a small diameter sphere that can focus the laser and fluorescent signal for use in a miniaturized or micro-optic application.

It will be appreciated that the detection assembly 620 may be movable. This may allow the detection assembly 600 to be retracted as the unit 60 is opened or closed to allow a user to easily place the container assembly 10 in the seat 600, and then place the laser 630 and detector 640 in position to interact with collection window 1203 of container assembly 10 when needed for use. It will be appreciated that the arrangement of the light source and detector are merely illustrative and that any arrangement that allows for focusing energy for excitation into the reaction chamber and detecting emitted signal from the reaction chamber may be used. For example, the positions of the light source and detector may be switched or they may be disposed at other locations.

The testing unit 60 may further include a CPU that controls its various components to perform a testing procedure, and a communications gateway, such as a Bluetooth or other wireless communications component. In some embodiment, the testing unit may perform self-diagnosis or calibration protocols, or may communicate with a remote system that directs the particular assay requirements and receives and analyzes data collected by the testing unit to determine a result. In some embodiments the system may utilize a handheld device, such as a user's smartphone, to route communications and may use an app on the device to report results to a user.

FIGS. 6B and 6C depict a testing container assembly 10A, including shroud 605A in position in a seat 600A in another exemplary embodiment of a testing unit 60A in accordance with the principles of the present disclosure. Seat 600A may be formed as a surface for receiving the base 1001 of the container assembly and may include alignment wall 601A, with the components of the testing unit 60A aligned to allow an assay to be performed. Seat 600A may include the various features and components discussed herein in connection with seat 600 of FIG. 6A. Testing unit 60A may also include a system for reducing or repositioning gas bubbles in reaction chamber 1200 to facilitate data collection.

Bubble reduction system 611 may include a rocker arm 6002, which extends from a driver assembly to a distal striking end 6003. In the depicted embodiment, the driver assembly includes a vibrator 6004 for actuating the rocker arm 6002 and a return spring 6005. Vibrator 6004 may be actuated by an electric motor (not shown) or as otherwise known in the art. It will be appreciated that any suitable driver assembly for actuating the rocker arm 6002 may be used.

The striking end 6003 of rocker arm 6002 may be disposed to reside underneath a portion of the base 1001 when the of the testing container assembly is in position in the seat 600. The seat 600A may include a recess through which rocker arm 6002 extends and/or an opening through which the striking end 6003 may emerge during use. Striking end 6003 may be positioned to reside underneath a portion of base 1001 nearer the distal end. The striking end may be positioned so that it does not engage with the reaction chamber 1200 when in use.

For use, a container assembly 10 or 10A along with a lid assembly 300 containing a plunger 400 may be provided to a user, as part of a testing kit. The components may be provided in a sealed package, such as a vacuum sealed envelope that maintains the sterility of components for use. Other materials, such as instructions or sample collection items may be provided in such a kit. In an exemplary embodiment of a PCR based test for the presence of a coronavirus in human saliva, a sealed container of a swishing solution and set of instructions may be provided.

A sample may then be collected. In the exemplary embodiment of a test for the presence of a coronavirus in human saliva, a user may swish the provided solution in their mouth and expectorate into the collection port 1000. Alternatively, a user may collect an anterior nares (nasal) specimen with a collection swab and swirl the swab in a solution either already dispensed in collection port 1000, or dispensed in a separate container that is then used to pour the specimen solution into the collection port 1000. The collection port 1000 may then be closed with the lid assembly 300, and as the lid assembly is secured, the distal end 4004 of the plunger may contact and dislodge the plug member 200, and the sample drawn into the sample preparation chamber 1100 around the recess 4006 by the vacuum. The testing container assembly 10 may be placed in the seat 600 of the testing unit 60 before or after the lid is secured. Where present, a longitudinal groove may allow venting of the pressure change as the plug member 200 is displaced.

Upon placement in the seat 60, the first temperature control device 610 is in conductive contact with the sample preparation chamber 1100 and may be actuated to perform or terminate desired sample preparation reactions. For example, the sample may be heated for a sufficient time at a sufficient temperature to deactivate proteases, nucleases and other enzymes that may naturally occur in saliva. If required pretreatments for a particular assay are desired, the reactants may be present in the sample preparation chamber 1100, as in dried form that is reconstituted by the sample, and appropriate conditions provided by the temperature control device 610. Once pretreatment is complete, the temperature control device 610 may be used to bring the sample to the appropriate temperature for further processing.

Once pretreatment is complete, the unit 60 may cause the frangible seal between the flexible sheet 1400 and the bottom surface 1004 to open, at least in the area of the reaction channel 1302, allowing the pretreated sample to flow along the reaction channel 1302 to the reaction chamber 1200. In the depicted embodiment, this may take place by advancing a piston through the top opening 3012 of the lid 300 into the bore 3010, where it contacts the upper end 4002 of piston 400 and advances distal end 4004 into the sample preparation chamber 1100, sealing the bore 1015 and displacing the fluid sample to pressurize and flex the flexible sheet 1400 to open reaction channel 1302.

Once a sufficient amount of pretreated sample has reached the reaction chamber 1200, the retractable sealing unit 618 may be extended and seal the sealing sheet 1400 to the bottom surface and thereby and isolate the reaction chamber 1200. In the depicted embodiment, such sealing may be performed to close the channel in the area generally indicated at 1114 (FIG. 1B). The diagnostic reactions can then be performed. The reactants for the particular reactions may be present in the reaction chamber 1200, as in dried form that is reconstituted by the sample, and appropriate conditions provided by the second temperature control device 614.

In one exemplary embodiment of a PCR based test for the presence of a coronavirus in human saliva, the required reactants may be present. In one embodiment in order to reduce required pretreatment steps, UNG enzyme may be present to reduce cross-contamination and false positives. Suitable thermal cycling for the PCR, may be provided to amplify the coronavirus genome specific regions. In some embodiments, fluorescent dyes linked to oligonucleotide probes which bind specifically to the amplified product during thermocycling may be used.

Where present, the bubble reduction system may be actuated to reduce any air or gas bubble in the reaction chamber. In the depicted embodiment, the drive may be actuated causing the rocker arm 6002 to vibrate, as vibrator 6004 is actuated, and the arm is restrained by return spring 6005. Striking end 6003 may repeatedly contact the base 1001, either for a set number of times, or set time providing energy to rupture or move any air bubbles in the reaction solution. Where the base is seated on an incline, any remaining bubbles may move towards a side of the chamber and away from a center of the detection window 1203. In embodiments, where the base 1001 may flex as the rocker arm is actuated, the sensor assembly 620 may be positioned away from the container assembly during the bubble reduction actuation.

When the reaction is sufficiently complete, detection may take place, in the exemplary embodiment, the laser 630 is used to provide energy to the reaction chamber at a first wavelength and the sensor 640 used to read emissions at various wavelengths on various channels. Where appropriate, sensor assembly 620 may be moved into position to allow detection to be performed.

In the exemplary embodiment, the CPU in the testing unit 60 may be used to controls its various components to perform the testing procedure. The communications gateway allows the CPU to communicate with a remote system that directs the particular assay requirements and receives and analyzes data collected by the testing unit to determine a result. In one exemplary embodiment, the unit 60 may be used in connection with a user's handheld mobile device, such as smartphone operating a software application, or app. The user places the unit 60 in communication through such device. In one example, instructions in the kit may contain a scannable code, or a scannable code may be present on the container assembly 10. The code is specific to the particular test and opens a control panel on the mobile device. The remote system provides control instruction to the unit to allow the procedure to be conducted and the data collected by the sensor 640 may be transmitted to the remote system. The remote system may then analyze the data, as by using the multiple channels to do data correction and standardization and determine the presence or absence of the amplified sequence product of interest. The result can then be transmitted back to the user in the app.

It will be appreciated that the data transmission may be encrypted, anonymized, or conducted in manner that complies with applicable privacy laws. Additionally, where a positive test result is required to be provided to a relevant authority, such as a local health department or the CDC, the remote system may do so.

It will be further appreciated that the systems, methods, and apparatus disclosed herein are not limited to single condition. Reactions used for detecting different analytes that require differing primers, enzymes and/or differing reaction conditions may be used. The remote system or the test unit itself may vary the particular conditions to conduct such tests. Multiple analytes can also be tested simultaneously in a single reaction chamber by use of multiple emission wavelengths detected by a multi-channel spectrometer. The portability and economy of the multi-channel spectrometer sensor and remote data analysis allow for the system to process such varying tests and for the unit to be operated in a residential or office setting instead of requiring a medical testing lab with trained personnel. It will be further appreciated that embodiments where the container assembly includes multiple reaction chambers and the testing unit contains the associated components for those reaction chambers are contemplated to allow for multiplex testing to be performed.

In another exemplary embodiment, systems, and containers in accordance with the present disclosure may be used with methods of detecting a nucleic acid in a sample. In such methods, a container or receptacle having a plurality of fluidly connected chambers including a sample preparation zone, and an amplification zone, with one or more sealable ports fluidly connecting the chambers may be provided. It will be appreciated that the e sealable ports may provide the only access from an exterior of the receptacle to the chambers. The sample may be introduced into the first chamber via a collection port, wherein the collection port has a bottom opening sealed by a plug member and when a lid assembly is secured to the collection port, to close the collection port, and the lid assembly includes a movable plunger assembly; the plug member may be contacted with the plunger to dislodge the plug member and allow the sample to enter a sample preparation chamber in fluid connection with the bottom opening.

Optionally, the sample may then be treated in the sample preparation chamber, as by heating to deactivate enzymes in the sample, or with reactants contained in the sample preparation chamber. A seal may be opened to allow the sample to flow from the sample preparation chamber to a reaction chamber. In some embodiments, this may be performed by advancing the plunger into the sample preparation chamber to pressure the sample and fracture the seal. The reaction chamber may then be sealed.

In the reaction chamber, nucleic acids in the sample may be mixed with PCR components including primers configured for amplifying one or more targets; and the target nucleic acids may be amplified.

A fluorescence emission signal from a fluorescent dye in the amplification zone may be detected, wherein fluorescence is excited by a laser diode, and emission is detected by a multi-channel spectrometer. A multi-channel spectrometer capable of processing at least 4 spectral channels in parallel in the wavelengths from approximately 350nm to 1000nm may be used. The fluorescence data may be communicated to a cloud or local CPU via a mobile phone or other user-controlled device or operating system. The data may then be analyzed, and the analysis result may be received from the cloud or local CPU by a mobile phone or other user-controlled device or operating system.

It will be appreciated that in such methods, the amplification zone may contain primers configured to amplify one or more nucleic acid targets that may be present in the sample. Such nucleic acid targets may originate from a pathogen, such as a virus, bacteria, and fungi. Some exemplary pathogenic viruses may include Coronavirus, Adenovirus, PIV1, PIV2, PIV3, RSV, Influenza A. Influenza B, Rhinovirus, and non-HRV Enterovirus. Some such Coronavirus targets may include 229E, NL63, OC43, and HKU1, MERS-CoV, SARS-CoV, and SARS-CoV-2. The methods may be optimized to detect a plurality of SARS-CoV-2 variants. In other embodiments, the nucleic acid target may be a human nucleic acid sequence. In further embodiments, targets may consist of both pathogen and human analytes, The amplification zone may be provided with dried amplification and detection reagents. In some embodiments, the amplification zone may be provided with means to perform melting curve analysis.

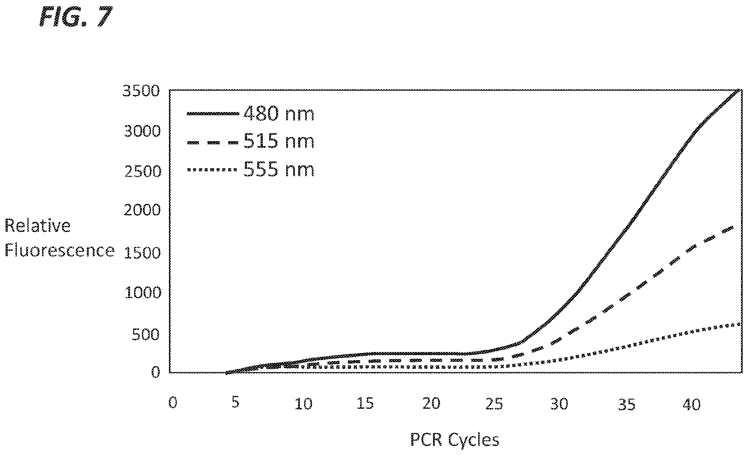

A first nonlimiting example of the detected results from an amplification method in accordance with the present disclosure is shown in FIG.7. A 50 bp fragment of a human polymorphism region (SNP RS1981929, chr2:47445309 + 47445358) was amplified in a testing container assembly that contained dry, premetered PCR reagents. In this example, 5 ng/µL human DNA of placental origin (Sigma-Aldrich, Saint Louis, MO) dissolved in 2 mL of water was introduced into the sample collection port 1000. Once plunger 400 was engaged, the DNA solution was introduced into the sample preparation chamber 1100. and subsequently to the reaction chamber 1200 which was filled to a volume of 65 µL. Primers 5'CGAGGTAGTGTATTATTAGTGGGAAG SEQ ID NO. 1. and 5'AGGGAGATGATGTAGCACTCA SEQ ID NO. 2 (IDT, Coralville, IA) in amounts to make final concentrations of 0.5 µM each once dissolved in the DNA solution, and Maverick Blue nucleic acid stain (Idaho Molecular, Inc., Salt Lake City, UT) to make a final concentration of 20 µM, were deposited in dry form to the bottom inner surface of the reaction chamber (bottom seal sheet 1400). Go-Taq DNA polymerase (0.13 U/µL, Promega, Madison, WI), 0.2 mM dNTPs, 4 mM MgCl₂, 50 mM Tris HCl pH 8.5, and 0.12 mg/mL BSA (all final concentrations) were deposited in dry form on the upper inner surface of the reaction chamber (detection window 1203). Amplification was performed at 90 °C for 0 seconds and 63 °C for 4 seconds for 45 cycles and monitored through the detection window 1203 in real time, using three wavelength channels (480 nm, 515 nm, and 555 nm). These three wavelength channels covered the range of fluorescence emission of Maverick Blue that was used to detect the amplification product. The testing unit utilized spherical lenses in front of a 450 nm laser diode as well as in front of the multi-channel spectrometer.

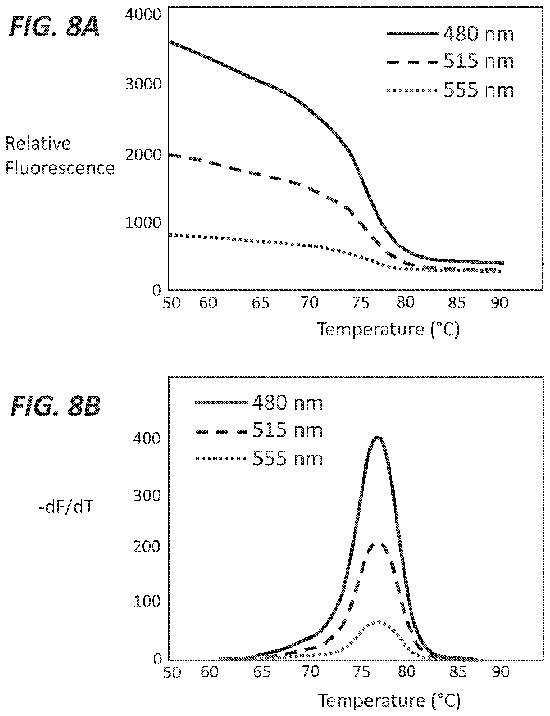

Following amplification, the sample was heated from 50 °C to 90 °C at a ramp rate of 0.35 °C/s, during which fluorescence was monitored in real time using the three channels (producing the results depicted in FIG. 8A) after which the data were converted to the negative derivative melting curves shown in FIG. 8B. The melting temperature of 77°C confirmed that the correct DNA fragment was amplified. All data processing took place in an external CPU that was in communication with the testing unit via Bluetooth.

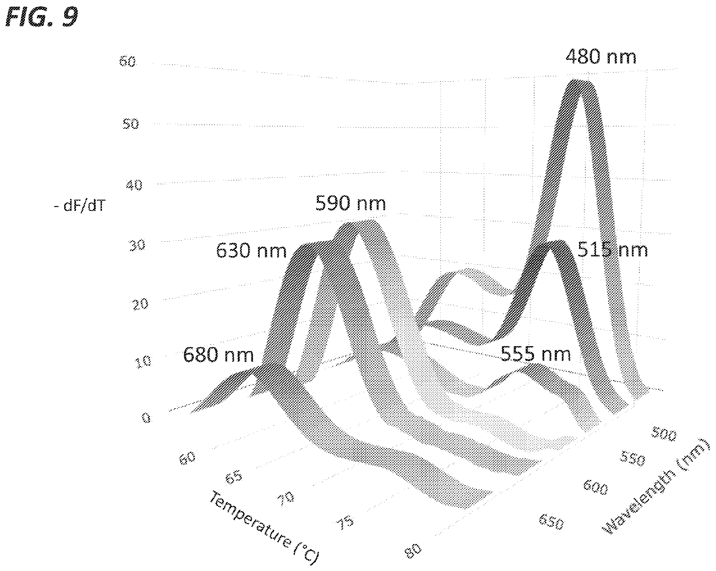

Another nonlimiting example is shown in FIG. 9 which depicts a melting curve analysis being performed in the testing container assembly using detection at six wavelengths 480 nm, 515 nm, 555 nm, 590 nm, 630 nm, and 680 nm. The testing unit has the same reagent and optical configuration as well as an external CPU as described above for FIGS. 7, 8A and 8B. Two synthetic double-stranded DNA fragments mimicking amplification products of high (75°C) and low (63°C) melting temperatures are melted in the reaction chamber 1200. The high melting temperature DNA is a double-stranded fragment created by hybridization of oligonucleotides with the sequence 5'CGGAATCTTGCACGCCCTCGCTCAGGCCTTCGTCACTGGTCCCGCCACC SEQ ID NO. 3 and its reverse compliment. The low melting temperature DNA is a double-stranded fragment created by hybridization of oligonucleotides 5'AGTGGAACCTCATCAGGAA SEQ ID NO. 4 and 5' CTCCTGATGAGGTTCCACCTGGTTT SEQ ID NO. 5 (where the underlined bases do not have complementary bases on the other strand). Both DNA fragments are stained with the double-stranded DNA binding dye Maverick Blue and are detected in the first three color channels (480 nm, 515 nm and 555 nm). The low melting temperature DNA fragment is further labeled with a carboxyrodamine (ROX) dye (which may be attached at the 5' end of AGTGGAACCTCATCAGGAA SEQ ID NO. 4) and is detected in three additional channels (590, 630 and 680 nm) enabled by fluorescent energy transfer from Maverick Blue.

While this disclosure has been described using certain embodiments, it can be further modified while keeping within its spirit and scope. This application is therefore intended to cover any variations, uses, or adaptations of the disclosure using its general principles. This application is intended to cover any and all such departures from the present disclosure as come within known or customary practices in the art to which it pertains, and which fall within the limits of the appended claims.

One non-limiting embodiment of the present invention will now be described by way of reference to the following clauses in which:
1. A system for conducting a biological assay, the system comprising:
   a testing container assembly comprising
      a sample collection port with a bottom opening that is sealed by a plug member,
      a sample preparation chamber in fluid connection with the bottom opening, and
      a reaction chamber;
   a lid assembly configured to close the sample collection port, the lid assembly including a movable plunger that contacts the plug member when the lid assembly is secured to the sample collection port; and a
   testing unit including a seat for receiving the testing container assembly, the testing unit comprising:
      a first temperature control device in conductive contact with the sample preparation chamber when the testing container assembly is placed in the seat,
      a second temperature control device in conductive contact with the reaction chamber when the testing container assembly is placed in the seat, and
      a detector assembly disposed to monitor conditions in the reaction chamber when the testing container assembly is placed in the seat.
2. The system of clause 1, wherein the sample collection port is formed as a cup shaped member disposed above the sample preparation chamber.
3. The system of clause 1, wherein the testing container assembly further comprises
   a base member body having an upper surface and an opposite bottom surface,
   a flexible bottom seal sheet adhered to portions of the bottom surface, with at least a portion of the lower end of the sample preparation chamber and at least a portion of the reaction chamber defined by space between the bottom surface and the bottom seal sheet.
4. The system of clause 3, wherein the reaction chamber includes a detection window formed in the upper surface of the base member body
5. The system of clause 4, wherein the detection window is formed from a flexible material.
6. The system of clause 3, wherein the sample preparation chamber is maintained under vacuum until the plug member is pushed from the bottom opening by the movable plunger as the lid assembly is secured.
7. The system of clause 3, further comprising a reaction channel extending between the sample preparation chamber and the reaction chamber, wherein the reaction channel is defined by space between the bottom surface and the bottom seal sheet.
8. The system of clause 7, wherein a frangible seal between the bottom sheet and the bottom surface of the base member seals the reaction channel until use.
9. The system of clause 8, wherein the bottom surface includes a stress riser that defines a point for opening of the frangible seal during use.
10. The system of clause 8, wherein the testing unit further comprises a plunger mechanism that contacts and advances the movable plunger to displace fluid in the sample preparation chamber and open the frangible seal.
11. The system of clause 8, wherein the testing unit further comprises a heat sealer for sealing a portion pf the bottom sheet to the bottom surface to close the reaction chamber.
12. The system of clause 1, wherein the detector assembly comprise a multi-channel spectrometer and a light source that is selected from a group consisting of a laser diode and a light-emitting diode.
13. The system of clause 12, wherein the detector assembly further comprises at least a first ball lens disposed to focus illumination of light from the light source onto the reaction chamber.
14. The system of clause 13, wherein the detector assembly further comprises a second ball lens disposed to focus emitted light from the reaction chamber to the multi-channel spectrometer.
15. A method of analyzing a sample, comprising
   placing a sample in a sample collection port in a container assembly, wherein the sample collection port has a bottom opening sealed by a plug member;
   securing a lid assembly to the sample collection port, to close the sample collection port, wherein the lid assembly includes a movable plunger assembly;
   contacting the plug member with the plunger assembly to dislodge the plug member and allow the sample to enter a sample preparation chamber in fluid connection with the bottom opening;
   opening a seal to allow the sample to flow from the sample preparation chamber to a reaction chamber;
   performing a reaction to amplify a biological marker of interest that may be present in the sample; and
   detecting the presence of amplified biological marker of interest in the reaction chamber.
16. The method of clause 15, wherein opening a seal to allow the sample to flow from the sample preparation chamber to the reaction chamber comprises advancing the movable plunger to displace fluid in the sample preparation chamber and open a frangible seal.
17. The method of clause 15. further comprising heating the sample in the sample preparation chamber to deactivate enzymes in the sample.
18. The method of clause 17, further comprising sealing the reaction chamber before performing the reaction to amplify a biological marker of interest that may be present in the sample.
19. The method of clause 15, wherein the biological marker of interest is at least one nucleic acid target and the reaction chamber contains primers configured to amplify one or more nucleic acid targets that may be present in the sample.
20. The method of clause 15, wherein the reaction chamber contains dried amplification and detection reagents therein.
21. The method according to clause 20, wherein the dried amplification and detection reagents are prepared by air drying.
22. The method according to clause 15, wherein detecting the presence of amplified biological marker of interest in the reaction chamber comprises detecting a fluorescence emission signal from a fluorescent dye,
23. The method according to clause 15, wherein detecting the presence of amplified biological marker of interest in the reaction chamber comprises performing melting curve analysis.
24. A method of detecting a nucleic acid in a sample, comprising:
   providing a receptacle having a plurality of fluidly connected chambers including
      a sample preparation zone,
      an amplification zone,
      one or more sealable ports fluidly connecting the chambers, the sealable ports providing the only access from an exterior of the receptacle to the chambers, and
   introducing the sample into the first chamber via a collection port, wherein the collection port has a bottom opening sealed by a plug member;
   securing a lid assembly to the collection port, to close the collection port, wherein flid assembly includes a movable plunger assembly;
   contacting the plug member with the plunger to dislodge the plug member and allow the sample to enter a sample preparation chamber in fluid connection with the bottom opening;
   opening a seal to allow the sample to flow from the sample preparation chamber to a reaction chamber;
   sealing the reaction chamber;
   mixing the nucleic acids in the sample with PCR components including primers configured for amplifying one or more targets;
   amplifying the target nucleic acids;
   detecting the fluorescence emission signal from a fluorescent dye in the amplification zone, wherein
      fluorescence is excited by a laser diode,
      emission is detected by a multi-channel spectrometer capable of processing at least 4 spectral channels in parallel in the wavelengths from approximately 350nm to 1000nm;
   communicating the fluorescence data to a cloud or local CPU via a mobile phone or other user-controlled device or operating system; and
   receiving the analysis result from the cloud or local CPU by a mobile phone or other user-controlled device or operating system.
25. The method according to clause 24, wherein the amplification zone contains primers configured to amplify one or more nucleic acid targets that may be present in the sample.
26. The method according to clause 25, wherein at least one nucleic acid target originates from a pathogen selected from the group consisting of virus, bacteria, and fungi.
27. The method according to clause 26, wherein the virus is selected from the group consisting of Coronavirus, Adenovirus, PIV1, PIV2, PIV3, RSV, Influenza A, Influenza B, Rhinovirus, and non-HRV Enterovirus.
28. The method according to clause 27, wherein the Coronavirus is selected from the group consisting of 229E, NL63, OC43, and HKU1, MERS-CoV, SARS-CoV, and SARS-CoV-2.
29. The method according to clause 28, wherein a plurality of SARS-CoV-2 variants is detected.
30. The method according to clause 25, wherein the nucleic acid target is a nucleic acid sequence of a human.
31. The method according to clause 25, wherein the amplification zone is provided with dried amplification and detection reagents therein.
32. The method according to clause 31, wherein the dried amplification and detection reagents are prepared by air drying.
33. The method according to clause 25, wherein the amplification zone is further provided with means to perform melting curve analysis.
34. The method according to clause 25, wherein the laser diode is used with a ball lens.
35. The method according to clause 25, wherein the spectrometer is used with a ball lens.

## Claims

1. A method of analyzing a sample, comprising
placing a sample in a sample collection port in a container assembly, wherein the sample collection port has a bottom opening sealed by a plug member;
securing a lid assembly to the sample collection port, to close the sample collection port, wherein the lid assembly includes a movable plunger assembly;
contacting the plug member with the plunger assembly to dislodge the plug member and allow the sample to enter a sample preparation chamber in fluid connection with the bottom opening;
opening a seal to allow the sample to flow from the sample preparation chamber to a reaction chamber;
performing a reaction to amplify a biological marker of interest that may be present in the sample; and
detecting the presence of amplified biological marker of interest in the reaction chamber.

2. The method of claim 1, wherein opening a seal to allow the sample to flow from the sample preparation chamber to the reaction chamber comprises advancing the movable plunger to displace fluid in the sample preparation chamber and open a frangible seal.

3. The method of claim 1, further comprising heating the sample in the sample preparation chamber to deactivate enzymes in the sample, wherein preferably the method further comprises sealing the reaction chamber before performing the reaction to amplify a biological marker of interest that may be present in the sample.

4. The method of claim 1, wherein the biological marker of interest is at least one nucleic acid target and the reaction chamber contains primers configured to amplify one or more nucleic acid targets that may be present in the sample.

5. The method of claim 1, wherein the reaction chamber contains dried amplification and
detection reagents therein, wherein preferably the dried amplification and detection reagents are prepared by air drying.

6. The method according to claim 1, wherein a) detecting the presence of amplified biological marker of interest in the reaction chamber comprises detecting a fluorescence emission signal from a fluorescent dye; or b) detecting the presence of amplified biological marker of interest in the reaction chamber comprises performing melting curve analysis.

7. A method of detecting a nucleic acid in a sample, comprising:
providing a receptacle having a plurality of fluidly connected chambers including
a sample preparation zone,
an amplification zone,
one or more sealable ports fluidly connecting the chambers, the sealable ports providing the only access from an exterior of the receptacle to the chambers, and
introducing the sample into the first chamber via a collection port, wherein the collection port has a bottom opening sealed by a plug member;
securing a lid assembly to the collection port, to close the collection port, wherein flid assembly includes a movable plunger assembly;
contacting the plug member with the plunger to dislodge the plug member and allow the sample to enter a sample preparation chamber in fluid connection with the bottom opening;
opening a seal to allow the sample to flow from the sample preparation chamber to a reaction chamber;
sealing the reaction chamber;
mixing the nucleic acids in the sample with PCR components including primers configured for amplifying one or more targets;
amplifying the target nucleic acids;
detecting the fluorescence emission signal from a fluorescent dye in the amplification zone, wherein
fluorescence is excited by a laser diode,
emission is detected by a multi-channel spectrometer capable of processing at least 4 spectral channels in parallel in the wavelengths from approximately 350nm to 1000nm;
communicating the fluorescence data to a cloud or local CPU via a mobile phone or other user-controlled device or operating system; and
receiving the analysis result from the cloud or local CPU by a mobile phone or other user-controlled device or operating system.

8. The method according to claim 7, wherein the amplification zone contains primers configured to amplify one or more nucleic acid targets that may be present in the sample.

9. The method according to claim 8, wherein at least one nucleic acid target originates from a pathogen selected from the group consisting of virus, bacteria, and fungi.

10. The method according to claim 9, wherein the virus is selected from the group consisting of Coronavirus, Adenovirus, PIV1, PIV2, PIV3, RSV, Influenza A, Influenza B, Rhinovirus, and non-HRV Enterovirus.

11. The method according to claim 10, wherein the Coronavirus is selected from the group consisting of 229E, NL63, OC43, and HKU1, MERS-CoV, SARS-CoV, and SARS-CoV-2, wherein preferably a plurality of SARS-CoV-2 variants is detected.

12. The method according to claim 8, wherein the nucleic acid target is a nucleic acid sequence of a human.

13. The method according to claim 8, wherein the amplification zone is provided with dried amplification and detection reagents therein, wherein preferably the dried amplification and detection reagents are prepared by air drying.

14. The method according to claim 8, wherein the amplification zone is further provided with means to perform melting curve analysis.

15. The method according to claim 8, wherein the laser diode is used with a ball lens or wherein the spectrometer is used with a ball lens.
